# EUROPÄISCHE PATENTSCHRIFT

(11) **EP 1 745 760 B1**
(45) Veröffentlichungstag und Bekanntmachung des Hinweises auf die Patenterteilung: **01.09.2010**
(21) Anmeldenummer: 06018549.3
(22) Anmeldetag: 12.05.2000
(51) Int. Cl.: A61C 13/00, A61C 5/10

(54) **Verfahren zur Herstellung eines Zahnersatzteiles, insbesondere aus beliebigen, auch biokompatiblen Werkstoffen und insbesondere mit Hilfe der CAD-CAM-Bearbeitungs-Technik**
Process for manufacturing customized implant-mounted tooth replacements and process for making a dental prosthesis, especially of any, also biocompatible material and especially with the aid of the CAD-CAM method
Procédé de fabrication personnalisé de protheses dentaires fixées sur implants et procédé de fabrication d'une prothèse dentaire, en particulier d'une quelconque matière, aussi en matière biocompatible, en particulier à l'aide de la CAD-CAM meulage et fraisage méthode

(30) Priorität: 21.06.1999 CH 115199
(43) Veröffentlichungstag der Anmeldung: 24.01.2007
(62) Teilanmeldung aus: 00110016.3
(73) Patentinhaber: Straumann Holding AG, 4052 Basel (CH)
(72) Erfinder: Traber, Tony, 4123 Allschwil (CH); Kappert, Heinrich, Prof. Dr. rer. nat., 79184 Gundelfingen (DE); Gläser, Rainer, 79104 Freiburg im Breisgau (DE)
(74) Vertreter: OK pat AG

(56) Entgegenhaltungen:
- US-A- 5 184 306
- US-A- 5 347 454
- US-A- 5 857 853
- US-A- 5 873 721

## Beschreibung

Die vorliegende Patentanmeldung ist eine Teilanmeldung aus der Europäischen Anmeldung Nr. 00 110 016.3 vom 12. Mai 2000, für welche die Priorität des Schweizer Patentgesuches Nr 1999 1151/99 vom 21. Juni 1999 beansprucht worden ist.

Gegenstand der Erfindung ist ein Verfahren zur Herstellung eines Zahnersatzteiles nach dem Oberbegriff des Patentanspruchs 1, wie z.B in US 587 371 offenbart.

Unter dem Begriff 'Zahnersatz' soll im Rahmen der vorliegenden Beschreibung ein Gebilde verstanden werden, das nicht nur den sichtbaren Teil eines Zahnes oder einer Zahngruppe ergänzt oder ersetzt sondern auch dessen oder deren Zahnwurzel, und das somit einen vollständigen Ersatz für einen oder mehrere Zähne eines Patienten bildet. Dieser Zahnersatz besteht im gefertigten Zustand aus mehreren ineinandergreifenden oder auch aneinander befestigten Bestandteilen. Unter dem Begriff 'Zahnersatzteil' soll dagegen im Rahmen der vorliegenden Beschreibung ein Gebilde verstanden werden, das auf einem Implantat oder einem präparierten Zahnrest abgestützt wird und das im Wesentlichen den Kieferknochen und das Zahnfleisch überragt; ein Zahnersatzteil kann insbesondere einen Bestandteil eines implantat-gestützten Zahnersatzes bilden.

Der erste Bestandteil des Zahnersatzes ist ein Implantat, welches im Kieferknochen des Patienten inseriert und dann auch als Implantat-Inserierung bezeichnet wird. Auf dieser Implantat-Inserierung werden später die weiteren Bestandteile des Zahnersatzes aufgebaut. Im Allgemeinen bildet die Implantat-Inserierung die Grundlage für einen Zahnersatz, der einen oder mehrere Zähne ersetzt; ein Zahnersatz, der einen Zahn ersetzen soll, wird auf einem einzigen Implantat abgestützt, und ein Zahnersatz, der mehrere Zähne ersetzen soll, wird im Allgemeinen auf mindestens zwei Implantaten abgestützt. Die Implantate sind dazu bestimmt, vom Zahnarzt im Kieferknochen eines Patienten implantiert bzw. inseriert zu werden, und sie werden dem Zahnarzt gebrauchsfertig und steril geliefert. Die Implantate können im Wesentlichen zylindrisch oder zulaufend ausgebildet sein und weisen die Form von Schrauben oder Stiften auf. An seinem coronalen Ende weist jedes Implantat ein erstes Positionierungselement auf. Die Implantate sind im eingegliederten Zustand des Zahnersatzes am Patienten nicht oder nur bedingt sichtbar. Auf dem Implantat kann ein Zahnersatzteil befestigt werden.

Bei der vorliegenden Erfindung tritt natürlicher Zahnrest an die Stelle künstlichen Implantates.

Der zweite Bestandteil des Zahnersatzes und ein Element des Zahnersatzteiles wird durch ein Verbindungsstück, das in der Fachsprache als Abutment bezeichnet wird, gebildet. An jedem Implantat bzw. Zahnrest wird ein Abutment befestigt. Die Abutments sind im eingegliederten Zustand des Zahnersatzes bzw. Zahnersatzteils am Patienten nicht oder nur bedingt sichtbar. Sie dienen dazu, die Implantate bzw. Zahnreste mit den weiteren Bestandteilen des Zahnersatzes zu verbinden. Jedes Abutment weist an seinem proximalen Ende ein zweites Positionierungselement auf, das zusammen mit dem ersten, am zugehörigen Implantat angeordneten Positionierungselement eine gegen Rotation sichernde Positionierungsvorrichtung bildet.

Der dritte Bestandteil des Zahnersatzes und ein weiteres Element des Zahnersatzteiles wird durch Verbindungselemente zur gegenseitigen Befestigung von Implantat bzw. Zahnrest und Abutment, allgemein Schrauben, gebildet. Auch diese Verbindungselemente sind im eingegliederten Zustand des Zahnersatzes bzw. Zahnersatzteils am Patienten nicht sichtbar. Die gegenseitige Verbindung von Implantat und Abutment ist im Allgemeinen reversibel.

Der vierte Bestandteil des Zahnersatzes und ein noch weiteres Element des Zahnersatzteiles wird als Gerüst, Brückengerüst oder Kappe bezeichnet. Er wird auf einem oder mehreren Abutments, im Allgemeinen mit Hilfe eines geeigneten Zementes oder Klebstoffes oder mittels einer Horizontal- oder Vertikalverschraubung gegen vertikale Verschiebungen gesichert beziehungsweise befestigt. Eine einzelne Kappe kann zum Ersatz eines Zahnes dienen und auf einem Abutment montiert werden. Ein Gebilde aus mehreren Kappen im Verbund mit einem Zwischenglied, als Pontic bezeichnet, kann aber auch als tragendes Element für eine Implantatbrücke zum Ersatz mehrerer Zähne bestimmt sein und auf mehreren Abutments montiert werden. Kappen sind zwar nach der Eingliederung des Zahnersatzes bzw. Zahnersatzteiles am Patienten nicht oder nur bedingt sichtbar, hingegen kann ihre Beschaffenheit einen gewissen Einfluss auf den ästhetischen Aspekt des Zahnersatzes bzw. Zahnersatzteils ausüben, wie weiter unten dargelegt werden wird.

Der fünfte Bestandteil des Zahnersatzes und ebenfalls ein Element des Zahnersatzteiles wird durch eine Verblendung gebildet, welche das Gerüst beziehungsweise das Brückengerüst beziehungsweise die Kappe ummantelt. Die Verblendung ist der einzige Bestandteil des Zahnersatzes bzw. Zahnersatzteils, der im eingegliederten Zustand am Patienten sichtbar ist. Die Verblendung ist dazu bestimmt, den sichtbaren Bereich des Zahnes zu ersetzen. Das Gerüst beziehungsweise das Brückengerüst beziehungsweise die Kappe einerseits und die Verblendung anderseits sind irreversibel miteinander verbunden.

Anstelle des Gerüstes beziehungsweise des Brückengerüstes beziehungsweise der Kappe sowie der Verblendung, welche - wie eben beschrieben - den vierten und den fünften Bestandteil des Zahnersatzes bilden, kann auch eine sogenannte Vollkrone verwendet werden; in diesem Falle umfasst der Zahnersatz nur vier Bestandteile, nämlich das Implantat, das Abutment, die Verbindungselemente und die Vollkrone.

Gerüst und Verblendung zusammen oder die Vollkrone allein bilden eine Einheit, die im Rahmen der vorliegenden Beschreibung auch als Suprakonstruktion bezeichnet wird. Die Suprakonstruktion ist reversibel oder irreversibel am Abutment befestigt; reversibel befestigte Suprakonstruktionen können bei Bedarf vom Abutment entfernt werden.

Der Zahnersatz bzw. das Zahnersatzteil soll bezüglich Funktionalität, Form und Aussehen dem zu ersetzenden natürlichen Zahn beziehungsweise den zu ersetzenden natürlichen Zähnen des Patienten möglichst ähnlich sein.

Wie schon erwähnt sollen unter dem Begriff eines Zahnersatzteiles im Rahmen der vorliegenden Beschreibung Gebilde verstanden werden, die dazu bestimmt sind, unmittelbar oder mittelbar auf Zahnresten befestigt zu werden. Somit fallen unter den Begriff von Zahnersatzteilen auf präparierten Zähnen befestigbare Teile wie beispielsweise kronen- und brückenartige Teile.

Die Herstellung von implantierbarem Zahnersatz kann in verschiedener Weise vor sich gehen, und auch der dabei entstehende Zahnersatz kann, wie oben beschrieben, unterschiedlich aufgebaut sein. Im Allgemeinen umfasst die Planung und Herstellung eines Zahnersatzes mehrere im Folgenden beschriebenen Verfahrensschritte, die teils vom Zahnarzt, teils vom Zahntechniker durchgeführt werden. Einige dieser Schritte entfallen, wenn nicht ein Zahnersatz sondern gemäss der vorliegenden Ausführung nur ein Zahnersatzteil erforderlich ist.

Der Aufgabenbereich des Zahnarztes beginnt mit der Erstellung eines Negativabdruckes, die auch als Kieferabformung bezeichnet wird. Die Erstellung eines Negativabdruckes bzw. einer Kieferabformungen ist ein Verfahrensschritt, bei welchem am Patienten Mass genommen wird, der aber am Patienten keine Spuren hinterlässt bzw. keine Veränderungen zur Folge hat. Aufgrund der Kieferabformung wird anschliessend durch den Zahntechniker mindestens ein Arbeitsmodell hergestellt; das Arbeitsmodell stellt die Situation im Kiefer des Patienten dar, in welchem der Zahnersatz zu integrieren ist. Der Zahnarzt liefert Angaben über die Anzahl der Implantate sowie gegebenenfalls weitere Angaben. Ferner inseriert der Zahnarzt die Implantate im Kiefer. Ein weiterer Negativabdruck wird nach der Inserierung der Implantate erstellt und liefert genauere Angaben für den Zahntechniker, die weiter unten beschrieben werden. Nach der vorgegebenen erforderlichen Heilphase, welche auf die Inserierung der Implantate im Kiefer des Patienten folgt, legt der Zahnarzt die Implantate beziehungsweise deren äusserste Bereiche frei. Die optimale Lage der Implantate ist für die Form der weiteren Bestandteile in Bezug auf Okklusion, Funktion und Ästhetik entscheidend; beim Inserieren der Implantate im Kiefer des Patienten ist es leider nicht immer möglich, die Implantate entsprechend den Idealvorstellungen bezüglich Okklusion, Funktion und Ästhetik optimal anzuordnen; die Folge davon ist, dass der Zahnersatz im Mund des Patienten bezüglich Okklusion, Funktion und Ästhetik häufig nicht optimal zu erstellen ist. Als letztes montiert der Zahnarzt auf den Implantaten nach einiger Zeit, während welcher diese einwachsen, die restlichen Bestandteile des Zahnersatzes.

Der Zahntechniker beginnt, wie oben erwähnt, mit der Erstellung eines Arbeitsmodells, des sogenannten Arbeitsmodells, das aufgrund des vom Zahnarzt gelieferten Negativabdruckes bzw. der Kieferabformung erstellt wird. Im weiteren Verfahren wird stets das Arbeitsmodell benutzt; auf dem Arbeitsmodell erfolgen die wesentlichen Arbeitsgänge der Herstellung des Zahnersatzes.

Der Zahntechniker arbeitet im Arbeitsmodell je nach herzustellendem Zahnersatz ein oder mehrere Manipulierimplantate, die auch als Modellanalog bezeichnet werden, ein. Die Manipulierimplantate sind kein Bestandteil des zu erstellenden Zahnersatzes sondern lediglich Hilfen zur Erstellung des Zahnersatzes. Das Modellanalog bildet die Grundlage für die Anfertigung der restlichen Bestandteile des Zahnersatzes. Die Position der Manipulierimplantate ist durch die vom Zahnarzt gelieferte Kieferabformung mit den Implantaten festgelegt. Die präzise Darstellung der Positionen der Implantate ist für die weitere Herstellung des Zahnersatzes entscheidend.

Auf jedes am Arbeitsmodell angeordnete Modellimplantat wird ein meist als Abutment bezeichnetes Verbindungsstück aufgebracht. Für die Beschaffung geeigneter Abutments gibt es mehrere Möglichkeiten. Es können vorkonfektionierte Abutments verwendet werden, was eine preisgünstigere und weniger zeitraubende Möglichkeit als die Herstellung individueller Abutments ist. Individuelle Abutments können durch einen Giessvorgang hergestellt werden, was aber mit mehreren Nachteilen behaftet ist; insbesondere ist es bei Brücken- und ähnlichen Konstruktionen schwierig, eine präzise Form sowie ein spannungsfreies Gebilde herzustellen; des Weiteren besteht beim Giessen die Gefahr von temperaturbedingten Werkstoffveränderungen. Individuelle Abutments können als Einzelteile individuell gefertigt werden, wobei sie individuell entweder aus einem Rohling oder aus einer Platte konfektioniert werden. Schliesslich können zur Herstellung individueller Abutments auch konfektionierte Abutments verwendet werden, die nachbearbeitet beziehungsweise individualisiert werden, wozu sie auf einer plattenartigen Haltevorrichtung befestigt werden. Die Herstellung individualisierter Verbindungskörper ist also in jedem Falle aufwendig.

Ein grosser Zeitaufwand entsteht bei der Herstellung implantatgetragener und zahnrestgetragener, das heisst basisteilgetragener Brücken, und zwar im Zusammenhang mit dem Einschieben der Suprakonstruktion auf die Basisteile bzw. Implantate oder Zahnreste und die Abutments. Während der Einschubvorgang bei der Herstellung eines kronenartigen Zahnersatzes, welcher sich über den Bereich nur eines Zahnes erstreckt, nicht mit allzu vielen Problemen behaftet ist, ist er bei der Erstellung eines brückenartigen Zahnersatzes, der sich über den Bereich mehrere Zähne erstreckt, bedeutend problematischer; da sich die Suprakonstruktion dabei mittelbar auf mehrere Basisteile, das heisst Implantate oder Zahnreste und unmittelbar auf mehrere Abutments abstützt. Die Basisteile, das heisst die Implantate oder Zahnreste, beziehungsweise deren Längsachsen sind im Allgemeinen nicht parallel sondern divergierend angeordnet und erfordern damit entsprechend divergierende Einschubrichtungen. Eine in einem Stück hergestellte Suprakonstruktion in Brückenform kann aber natürlich nur in einer einzigen Einschubrichtung eingegliedert werden, das heisst die Einschubrichtungen auf den Abutments müssten parallel sein, was sich derzeit nur unter erschwerten Bedingungen realisieren lässt. Die Eingliederung wird bei grossen Abweichungen unmöglich, oder erst dann möglich, wenn eine manuelle, grossen Aufwand erfordernde Nachbearbeitung stattgefunden hat. Bei kleineren Abweichungen ist es noch möglich, die Eingliederung im Munde des Patienten vorzunehmen, allerdings nur unter Erzeugung von Spannungen an den Implantaten bzw. Zahnresten und/oder an den Abutments und/oder an der Suprakonstruktion.

Mit den herkömmlichen Verfahren, die mit vielen möglichen Fehlerquellen behaftet sind, ist es derzeit ausserordentlich schwierig, eine spannungsfreie Konstruktion mit nicht veränderten Werkstoffeigenschaften des Zahnersatzes zu erhalten.

Aufgabe der Erfindung ist es daher, ein verbessertes Verfahren zur Herstellung eines individuellen Zahnersatzteiles, das heisst eines individuellen Zahnersatzteiles aus einem oder mehreren Elementen, das auf mindestens einem Zahnrest, das heisst auf Resten von mindestens zwei Zähnen montierbar ist, vorzuschlagen.

Die Lösung dieser Aufgabe erfolgt für die Herstellung des Zahnersatzteiles durch die Merkmale des kennzeichnenden Teils des Anspruchs 1.

Vorteilhafte Weiterbildungen des erfindungsgemässen Verfahrens werden durch die abhängigen Patentansprüche **2** bis 11 definiert.

Zur Herstellung eines Zahnersatzes, der ein Implantat umfasst, wird wie bei herkömmlichen Verfahren vom Zahnarzt ein Negativabdruck bzw. eine Negativabformung des Kiefers oder Kieferbereiches, in welchen der anzufertigende individuelle Zahnersatz später zu liegen kommt, hergestellt, um Informationen bezüglich der Lage der Basis beziehungsweise des Implantates oder der Implantate zu bekommen. Vom Zahntechniker wird aufgrund dieser Negativabformung ein Arbeitsmodell hergestellt. Anschliessend wird auf dem Arbeitsmodell eine Manipulierimplantatanordnung angebracht, die bezüglich ihrer Lage den Implantaten im Mund des Patienten entspricht. Die Manipulierimplantatanordnung kann aus einem oder mehreren Manipulierimplantaten bestehen, entsprechend der Anzahl der tatsächlichen Implantate. Die Manipulierimplantate ragen nicht oder kaum aus dem Arbeitsmodell, ebenso wie die Implantate nicht oder kaum aus dem entsprechenden Kieferbereich des Patienten ragen. Als nächstes wird daher auf jedem Manipulierimplantat provisorisch ein Hilfselement angebracht. Diese Hilfselemente ragen aus dem Arbeitsmodell und bilden Hilfselemente bzw. Vermessungsteile, da sie die Einsatztiefe, die Längsachsenrichtung und die Winkelstellung der Manipulierimplantate im Arbeitsmodell und damit auch die Implantat-Tiefe, die Implantat-Achsrichtung und die Implantat-Winkellage im Mund des Patienten wiedergeben. Mit Hilfe dieser Hilfselemente oder Vermessungsteile lassen sich anschliessend Daten der Geometrie des Arbeitsmodells erfassen, und daraus kann auf die genaue Lage der Implantate im Kieferbereich des Patienten geschlossen werden. Aus den einmal erfassten Daten lassen sich weitere Daten ermitteln, die für die vollautomatisierte Herstellung sowie für die Ermittlung der Einschubrichtung der weiteren Bestandteile des individuellen Zahnersatzes benötigt werden; insbesondere ist es möglich, die Bestandteile von mehrere Implantate erfassendem Zahnersatz so herzustellen, dass die Eingliederung in einer einzigen Einschubrichtung, die auch als parallele Einschubrichtung bezeichnet wird, erfolgen kann. Dadurch wird in jeder Beziehung eine bisher unerreichte Präzision gewährleistet.

Die erfindungsgemässe Herstellung eines Zahnersatzteiles nach dem neuen Verfahren ist im wesentlichen gleich wie die Herstellung eines Zahnersatzes, mit Ausnahme der Verfahrensschritte, die sich lediglich auf die Implantate beziehen, da Zahnersatzteile auf vorhandene Restzähne oder auf als vorhanden vorausgesetzte Implantate abgestützt sind.

Weitere Einzelheiten und Vorteile der Erfindung werden im folgenden anhand von Ausführungsbeispielen und mit Bezug auf die Zeichnung beschrieben. Es zeigen:
- **Fig. 1**: einen Zahnersatz , in einem die Längsachse des Zahnersatzes enthaltenden Schnitt;
- **Fig. 2**: einen Kieferbereich eines Patienten, vor der Implantierung der Implantate, in einem Schaubild;
- **Fig. 3**: ein Arbeitsmodell des in ***Fig. 2*** dargestellten Kieferbereiches, ausschnittsweise, mit zwei divergent stehenden Implantaten und einem Hilfselement pro Imp- lantat, vereinfacht dargestellt, in einem Schnitt;
- **Fig. 4**: zeigt Standardabutments wo die gemeinsame Einschubrichtung nicht gegeben ist, was dem Stand der Technik entspricht;
- **Fig. 5**: den in ***Fig.* 2** dargestellten Kieferbereich des Patienten mit einem aufgrund des Arbeitsmodells der ***Fig. 3*** und der ***Fig. 4*** hergestellten Zahnersatz, in gleicher Darstellung wie ***Fig. 3*** und ***Fig. 4****;*

Die Figuren 6 - 9 beschreiben die Herstellung von Zahnersatz, dienen aber auch zur Erläuterung der Herstellung von Zahnersatzteilen.
- **Fig. 6A**: eine Platte zur Aufnahme von Abutment- Rohteilen, auf welcher mehrere Abutments während ihrer Bearbeitung fixierbar sind, in Draufsicht;
- **Fig. 6B**: die in ***Fig. 6A*** dargestellte Platte mit einem auf der Platte befestigten Abutment-Rohteil und mit drei nicht befestigten Abutments sowie der zugehörigen Befestigungsschrauben, in einem Schnitt senkrecht zur Plattenhauptebene;
- **Fig. 7A**: eine Platte, aus welcher anatomisch korrekte Abutments korrekt fräsbar sind, in Draufsicht;
- **Fig. 7B**: die in ***Fig. 7A*** dargestellte Platte, in einem Schnitt senkrecht zur Plattenhauptebene;
- **Fig. 7C**: eine weitere Platte, aus der Abutments herstellbar sind, in gleicher Darstellung wie ***Fig. 7A******;***
- **Fig. 7D**: die in ***Fig. 7C*** dargestellte Platte, in welcher die Formen der herzustellenden Abutments in gestrichelten Linien dargestellt sind, von der Seite;
- **Fig. 8A**: vier aus einer Platte hergestellte Abutments in derjeni gen Lage, die sie in der Platte eingenommen haben,
wobei die Platte gestrichelt dargestellt ist, in Draufsicht;
- **Fig. 8B**: die in ***Fig. 8A*** dargestellten Abutments, wobei die Platte, aus welcher sie hergestellt sind, gestrichelt dargestellt ist, von der Seite;
- **Fig. 9A**: vier aus einer Platte hergestellte aus der Integralteile, umfassend Abutments, Gerüste und Verblendungen, in derjenigen Lage, die sie in der Platte eingenommen haben, wobei die Platte gestrichelt dargestellt ist, in gleicher Darstellung wie ***Fig. 8A******;***
- **Fig. 9B**: die in ***Fig. 9A*** dargestellten Integralteile, in gleicher Darstellung wie ***Fig. 8B*****;**
- **Fig. 10**: ein Blockdiagramm zur Verdeutlichung des Verfahrens zur Herstellung eines Zahnersatzes; und
- **Fig. 11**: ein Schema zur Erläuterung unterschiedlicher Herstellungsmethoden und ―phasen von Zahnersatzteilen.

Die in der vorliegenden Beschreibung behandelte Ausführung betrifft, wie weiter oben mehrfach erwähnt, ein Verfahren zur Herstellung eines individuellen Zahnersatzteiles, das heisst eines individuellen Zahnersatzteiles aus einem oder mehreren Elementen, das auf natürlichem Zahnmaterial montierbar ist. Die einzelnen Bestandteile bzw. Elemente des Zahnersatzteiles sind im Wesentlichen die gleichen wie die Bestandteile eines Zahnersatzes, jedoch ohne Implantate, an deren Stelle hier Zahnreste benutzt werden. Das Zahnersatzteil kann daher im Prinzip gleich hergestellt und montiert werden wie oben beschrieben.

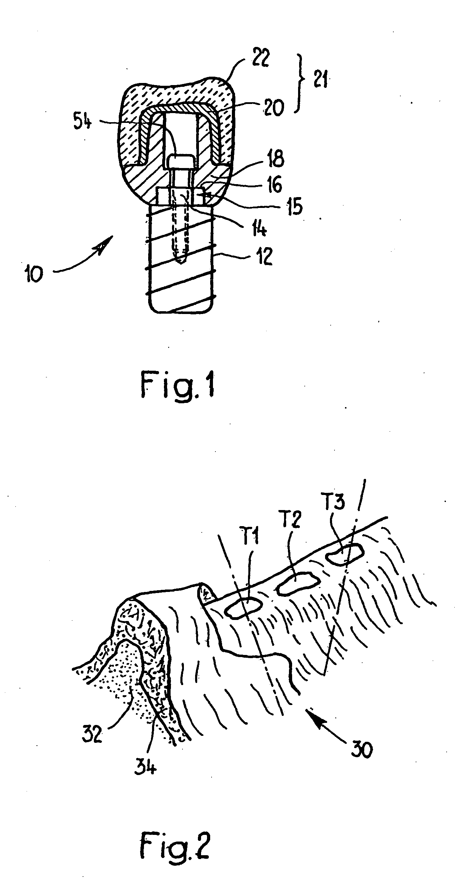

In **Fig. 1** ist ein vollständiger individueller Zahnersatz **10** dargestellt. Der individuelle Zahnersatz **10** umfasst ein Implantat **12**, welches ein Basisteil für weitere Bestandteile, , das heisst für das eigentliche Zahnersatzteil, bildet. Das Basisteil ist, bei einem in der Stammanmeldung beanspruchten Verfahren, ein Implantat, das hier als Schraube ausgebildet ist. Das Implantat **12** ist gemäss der Stammanmeldung dazu bestimmt, als künstlicher Ersatz für die Zahnwurzel bzw. den Zahnrest im Kieferknochen des Patienten befestigt zu werden, wobei beim Verfahren gemäss der vorliegenden Teilanmeldung anstelle des künstlichen Basisiteils in Form dese Implantats ein natürliches Basisteil in Form eines Zahnrestes vorhanden ist.

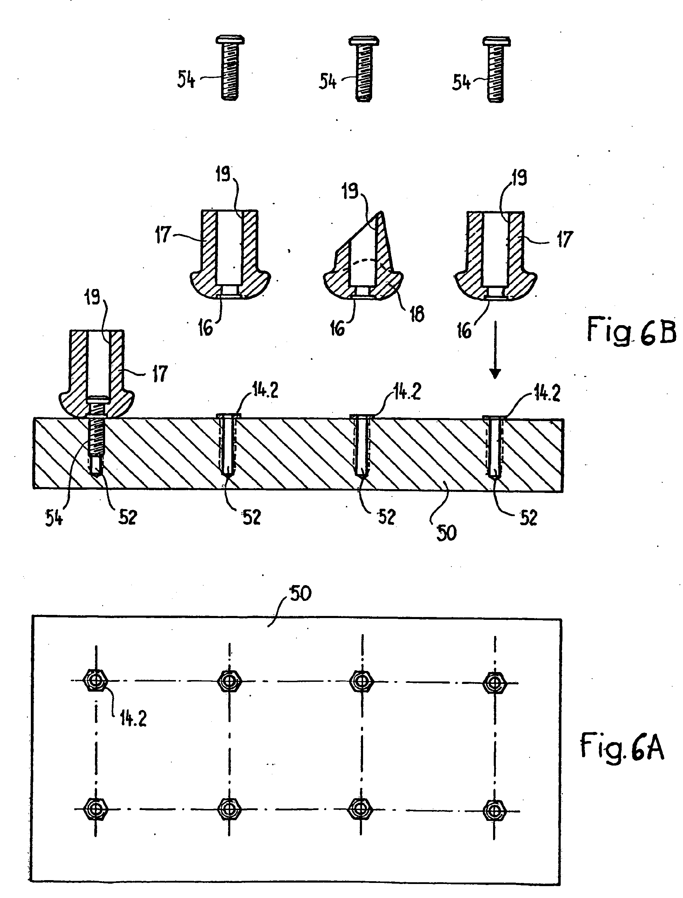

Am in Fig. 1 oberen Ende weist das Basisteil 12 ein erstes Positionierungselement **14** einer Positionierungsvorrichtung 15 auf. Ein zweites, komplementär zum ersten ausgebildetes Positionierungselement 16 derselben Positionierungsvorrichtung 15 ist an einem Abutment **18** angeordnet und in **Fig. 6A, 6B** dargestellt. Ebenfalls ist in **Fig. 6B** eine Durchgangsbohrung 19, die auch als Montagekanal bezeichnet wird, sichtbar, die zur Aufnahme eines Befestigungselementes wie einer Schraube **54** vorgesehen ist, mittels welcher das Abutment **18** auf dem Basisteil 12 befestigt ist.. Auf dem Basisteil 12 ist gemäss Fig. 1 ein Gerüst 20 aufgebaut, das auch als Kappe bezeichnet wird. Für das Gerüst 20 können unterschiedliche Werkstoffe und Herstellungstechniken verwendet werden, wie dies weiter unten beschrieben wird. Auf dem Gerüst 20 ist eine Verblendung 22 befestigt, welche zusammen mit dem Gerüst 20 eine im Rahmen der vorliegenden Beschreibung eine als Suprakonstruktion **21** oder Mesiostruktur **21** bezeichnete Einheit des individuellen Zahnersatzes 10 und somit des Zahnersatzteiles bildet. Die Verblendung 22 stellt im Wesentlichen den Ersatz für den sichtbaren Teils des Zahnschmelzes dar. Fig. 1 dient nur zur Erläuterung der Bestandteile, aus welchen das im Munde des Patienten angebrachte individuelle Zahnersatz 10 aufgebaut ist, nicht aber zur Beschreibung des Vorgehens beim Aufbau beziehungsweise bei der Montage des individuellen Zahnersatzteiles.

Fig. 2 stellt ausschnittsweise einen Kieferbereich 30 mit einem Kieferknochen 32 und Zahnfleisch 34 eines Patienten dar, der mit einem brückenartigen individuellen Zahnersatz 10 zu versehen ist, und zwar an den Stellen T1, T2, T3, wo ursprünglich drei benachbarte Zähne angeordnet waren. An diesem Kieferbereich 30 wird vom Zahnarzt eine nicht dargestellte Kieferabformung in Form eines Negativmodells beziehungsweise Negativabdruckes angefertigt. Ferner wird der Zahnarzt an den Stellen T1 und T3 je ein in Fig. 2 nicht dargestelltes lmplantat 12 implantieren beziehungsweise inserieren und auf diesen beiden Implantaten 12, sobald sie ossio-integriert sind, die weiteren Bestandteile des individuellen Zahnersatzes 10 befestigen, die vom Zahntechniker hergestellt werden. Ausserdem liefert der Zahnarzt Informationen bezüglich der Lage der beiden Implantate 12 und der erwünschten Ausbildung der weiteren Bestandteile des individuellen Zahnersatzes 10.

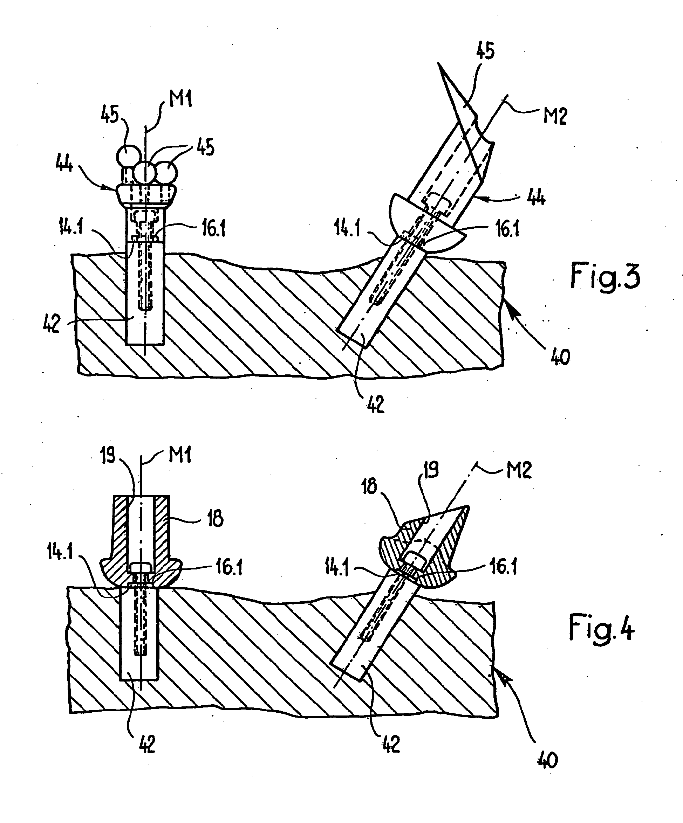

Aufgrund des Negativabdruckes beziehungsweise der Kieferabformung des Zahnarztes werden vom Zahntechniker ein oder mehrere Arbeitsmodelle, insbesondere ein in **Fig. 3** ausschnittsweise wiedergegebenes Arbeitsmodell **40,** hergestellt. Das Arbeitsmodell **40** dient dem Zahntechniker als Grundlage zur Herstellung des individuellen brückenartigen Zahnersatzteiles, das die drei ursprünglich bei **T1, T2** und **T3** vorhandenen gewesenen Teile der Zähne ersetzen soll. Das brückenartige individuelle Zahnersatzteil gemäss der vorliegenden Teilanmeldung weist als Basisteil 12, wie schon erwähnt, nicht zwei Implantate 12 sondern zwei Zahnreste auf. Am Arbeitsmodell **40** werden nun zwei Manipulierelemente **42** befestigt, und zwar an Stellen bzw. mit Längsachsen **M1** beziehungsweise **M2,** die den Stellen bzw. Hauptrichtungen **T1** und **T3** von zweien der zu ersetzenden drei Zähne möglichst genau entsprechen.

Alternativ kann das Arbeitsmodell auch aufgrund einer Abformung des die Basisteile 12 enthaltenden Kieferbereiches 31.1 erhalten werden, wobei dann im Allgemeinen keine Manipulierelemente **42** benötigt werden.

In **Fig. 3** ist diejenige Herstellungsphase des individuellen Zahnersatzteiles dargestellt, in welcher auf jedem der Manipulierelemente **42** ein provisorisch zugehöriges Hilfselement beziehungsweise Vermessungsteil **44** angeordnet ist; diese Hilfselemente beziehungsweise Vermessungsteile **44** gehören nicht zum definitiven Zahnersatzteil. Die Hilfselemente beziehungsweise Vermessungsteile **44** weisen Enden bzw. Markierungen **45** auf, anhand derer sich ihre Stellung und winkelmässige Anordnung erkennen lässt. Die beiden Manipulierelemente **42** beziehungsweise ihre Längsachsen stehen häufig weder parallel noch liegen sie in einer gemeinsamen Ebene, sondern sie nehmen divergierende Stellungen ein.

An jedem Manipulierelement **42** wird in einer späteren Herstellungsphase des individuellen Zahnersatzteils gemäss **Fig. 4** ein Abutment **18** provisorisch montiert. Das Abutment **18** weist an seinem dem Basisteil zugewandten Ende das Positionierungselement **16** auf, welches letztlich dazu bestimmt ist, mit dem am oberen beziehungsweise äusseren Ende des Basisteils vorhandenen komplementären Positionierungselement **14** zusammenzuwirken, um mit dem letzteren zusammen die zur Rotationsverhinderung dienende Positionierungsvorrichtung **15** zu bilden. Entsprechend weisen das Manipulierelement **42** ein Positionierungselement **14.1** und das Hilfselement beziehungsweise Vermessungsteil **44** ein Positionierungselement **16.1** auf, welches eine relative Rotation verhindert und die Position sichert.

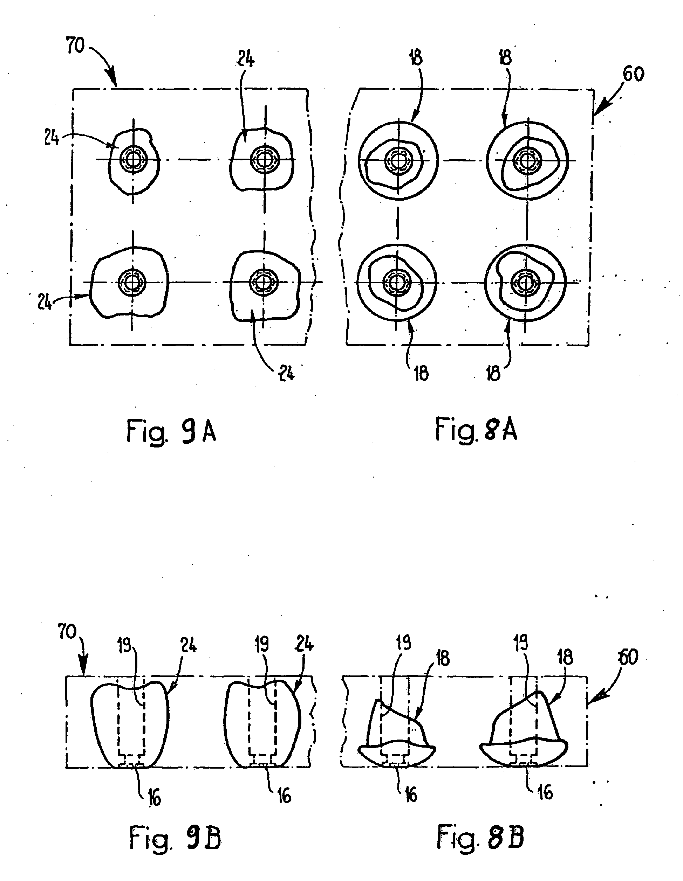

Der entsprechende Bereich des Arbeitsmodells **40** mit den sichtbaren Teilen der Hilfselemente beziehungsweise Vermessungsteile **44,** jedoch ohne die Abutments 18, in dreidimensionaler Konfiguration wird mittels einer dreidimensionalen Erfassungsanlage, vorzugsweise mittels eines Scanners **SCAN,** der schematisch in **Fig. 9** wiedergegeben ist, erfasst und die entsprechenden Werte in einer Speichereinheit einer EDV-Anlage **EDV** gespeichert beziehungsweise gelagert. Die durch den Scan-Vorgang ermittelten Werte werden anschliessend mit Hilfe der EDV-Anlage EDV, welche auch in einer CAD- beziehungsweise CAM- beziehungsweise CAD/CAM-Anlage CC integriert sein kann, in Basis-Daten **BAD** umgesetzt. Diese Basis-Daten BAD werden für alle weiteren Berechnungen und automatisierten Bearbeitungsvorgänge benutzt.

Aus den Basis-Daten **BAD** werden Basisteil-Daten **ID** ermittelt, welche aufgrund der Lage der Hilfselemente beziehungsweise Vermessungsteile 44 im Arbeitsmodell **40** die Lage der Basisteile im Kieferbereich **30** des Patienten beschreiben. Aus den Basis-Daten **BAD,** die die Lage der Hilfselemente **44** definieren, lassen sich somit die Neigung und die Stellung der Basisteile selbst sowie insbesondere die Ausrichtung des Positionierungselements **14,** beispielsweise Hex, etc., ermitteln.

Aus den Implantat-Daten ID sowie aus weiteren Daten, die auch durch ein Wax-Up gewonnen werden können, lassen sich Abutment-Daten **AD** gewinnen, welche die Formgebung und die beabsichtigte Lage des beziehungsweise der für den jeweiligen Fall erforderlichen Abutments **18** definieren. Aufgrund dieser Abutment-Daten **AD** wird ein geeigneter Verfahrensablauf bzw. ein geeignetes Flussverfahren gewählt. Anschliessend wird in der CAD/CAM-Anlage CC das individuelle Abutment **18** durch spanabhebende Bearbeitung wie Schleifen und/oder Fräsen hergestellt, wobei als Rohmaterial entweder ein einzelnes Abutment-Rohteil oder eine Platte aus Abutmentmaterial verwendet wird, wie dies weiter unten beschrieben wird. Die vollautomatisierte Fertigung der Abutments **18** auf der CAD/CAM-Anlage CC gewährleistet nicht nur die geometrische Passgenauigkeit sondern auch die Einhaltung der vorbestimmten Materialeigenschaften, da diese während des Bearbeitungsprozesses keinen unvorhersehbaren mechanischen und thermischen Einflüssen ausgesetzt werden.

Auf das Verfahren zur Herstellung der individuellen Abutments wird weiter unten genauer eingegangen.

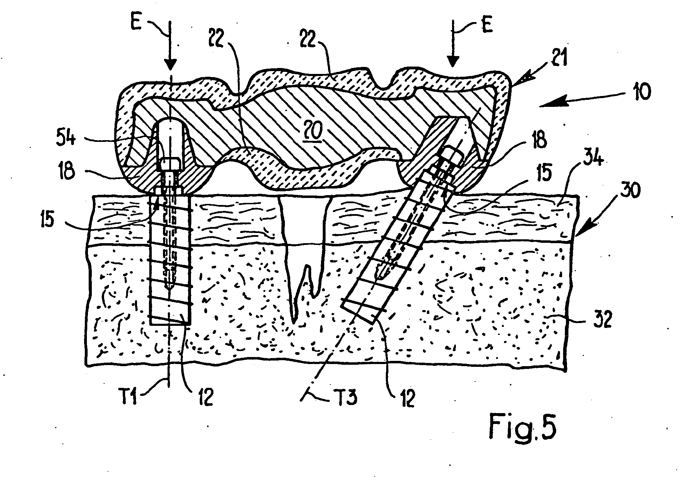

Werden gemäss den **Fig. 2** bis **5** mehrere Abutments **18** für den individuellen Zahnersatz **10** benötigt, der in fertigem, am Patienten montiertem Zustand in **Fig. 5** dargestellt ist, so müssen sie derart konfiguriert sein, dass die tiefen-, richtungs-, und winkelmässigen Abweichungen zwischen den Basisteilen **12** kompensiert werden, damit anschliessend die Suprakonstruktion **21,** bestehend aus dem Gerüst **20** und der Verblendung **22,** in einer gemeinsamen Einschubrichtung montiert werden kann, ohne dass Spannungen in den Basisteilen, in den Abutments **18** und in der Suprakonstruktion **21** entstehen. Zu diesem Zwecke kann die EDV-Anlage **EDV** aus den Basis-Daten **BAD** nicht nur die eigentlichen Abutment-Daten **AD** sondern auch Einschub-Daten **ED** ermitteln, welche die Einschubrichtung, insbesondere bei mehreren Basisteilen die gemeinsame Einschubrichtung, definieren, in welcher schliesslich die Suprakonstruktion **21** auf die Abutments **18** geschoben wird.

**Fig. 6** zeigt einen Kieferbereich mit zwei Implantaten **12,** an deren Stelle bei der vorliegenden Erfindung als Basisteile zwei Zahnreste vorhanden sind, und mit je einem darauf befestigten Abutment **18,** sowie mit einer Standardplatte **46** für eine Brückenkonstruktion.

Die EDV-Anlage kann aus den Basis-Daten **BAD** ferner Gerüst-Daten **GD** bestimmen, da im Wesentlichen die innere seitliche Fläche des Gerüstes **20** auf die äussere bzw. obere Fläche der Abutments **18** passen muss, damit das Gerüst **20** spannungsfrei und haltbar auf den Abutments **18** befestigt werden kann. Dies bedeutet nicht, dass die beiden genannten Flächen kongruent sein müssen, da zu ihrer gegenseitigen Befestigung Zement verwendet wird, für welchen ein geringer Spalt freizuhalten ist. Auch das Gerüst **20** oder die kronen- oder brückenartige Suprakonstruktion **21** kann mit Hilfe der CAD/CAM-Anlage CC hergestellt werden, wobei sich dieselben Vorteile ergeben, die weiter oben bezüglich der Herstellung der Abutments **18** erwähnt worden sind.

Das individuelle Zahnersatzteil, jedoch ohne die ihn später im Kiefer des Patienten tragenden Basisteile bzw. Zahnreste, wird provisorisch am Arbeitsmodell **40** aufgebaut; anstelle der Zahnreste werden hierbei die Manipulierbasisteile **42** benutzt. Anschliessend erfolgt der Transfer der Abutments **18** sowie des Gerüstes **20** und der Verblendung **22** in den Kieferbereich **30** des Patienten; dieser Kieferbereich mit dem fertigen individuellen Zahnersatzteil ist in **Fig. 5** dargestellt. Die Abutments **18** werden dabei auf den Basisteilen bzw. Zahnresten mittels der Positionierungselemente **16** positioniert und mittels Schrauben befestigt. Auf die Abutments **18** wird dann die Suprakonstruktion **21,** mit dem Gerüst **20** und der Verblendung **22,** geschoben, und zwar in der Einschubrichtung E, die für die verschiedenen Abutments **18** parallel ist.

Wesentlich für die Präzision des individuellen Zahnersatzteiles bei der Herstellung ist, dass zu allen Zwecken, zu denen Daten benutzt werden müssen, stets auf dieselben, durch einen einzigen Scan-Vorgang ermittelten Basis-Daten **BAD** zurückgegriffen wird, aus welchen sich unmittelbar und mittelbar alle weiteren Daten wie zum Beispiel Abutment-Daten **AD,** Fixierungsdaten **FD,** Gerüstdaten **GD,** Verblendungsdaten **VD** ableiten lassen, um sie für die Konfiguration und Bearbeitung der verschiedenen Bestandteile in der CAD/CAM-Anlage CC zu benutzen. Ebenso wichtig ist es, dass keine manuellen Verfahrensschritte erforderlich sind, welche die präzise Position der Basisteile **12** und die Lage, Form und Materialbeschaffenheit der Abutments **18** sowie die Lage der Suprakonstruktion **21** beeinflussen. Die Basis-Daten **BAD** ermöglichen ein systematisches, kohärentes Verfahren zum Aufbau des individuellen Zahnersatzes.

Im folgenden wird auf Einzelheiten der Herstellung des Verbindungsteile bzw. Abutments **18** und auf Abutment-Rohteile **17** eingegangen. Als Abutment-Rohteil **17** für ein einzelnes Abutment **18** wird ein Rohling oder ein Halbfabrikat verwendet, welches vom Hersteller dazu bestimmt ist, nachgearbeitet und dadurch individualisiert zu werden.

Eine Bibliothek in der Speichereinheit der EDV-Anlage **EDV** kann die Daten von erhältlichen vorgefertigten Abutment-Rohteilen **17** enthalten, wobei es sich hierbei nicht nur um Abutment-Rohteile **17** aus einem einzigen Material oder von einem einzigen Hersteller handeln muss. Aus diesen erhältlichen vorgefertigten Abutment-Rohteilen **17** sucht die EDV-Anlage **EDV** nun für jedes Implantat **12** das zugehörige Abutment-Rohteil **17** aus. Im Allgemeinen handelt es sich beim jeweils geeignetsten Abutment-Rohteil **17** um dasjenige, aus welchem sich das passende Abutment **18** mit dem geringsten Bearbeitungsaufwand fertigen lässt. Im weiteren bestimmt die EDV-Anlage **EDV** alle geometrischen Bearbeitungsdaten sowie beispielsweise Schnitt- und Vorschubgeschwindigkeiten, Menge des benötigten Kühlmittels und andere Daten, nach welchen die Bearbeitungseinheit der CAD/CAM-Anlage CC das Abutment **18** aus dem Abutment-Rohteil **17** herstellt.

Alternativ kann als Abutment-Rohteil **17** auch ein konfektioniertes Norm-Abutment verwendet werden, das zwar vom Hersteller als fertiges Abutment angeboten wird und das ohne weitere Bearbeitung zur sofortigen Verwendung bestimmt wäre; ein solches Norm-Abutment muss aber zur Erzeugung eines optimalen individuellen Zahnersatzes **10** gemäss der Stammanmeldung oder Zahnersatzteils gemäss dem vorliegenden Teilgesuch dennoch weiterbearbeitet und dadurch individualisiert werden.

Einzelheiten zur Herstellung der Abutments **18** aus den Abutment-Rohteilen **17** werden in **Fig. 6A** und **6B** gezeigt. Die Abutment-Rohteile **17** werden während ihrer Bearbeitung auf einer Vorrichtung befestigt. Es hat sich aber als günstig erwiesen, mehrere Abutment-Rohteile auf einer gemeinsamen Vorrichtung zu befestigen. Diese Vorrichtung in Form einer Platte **50** ist in **Fig. 6A** in Aufsicht dargestellt. Auf dieser Platte **50** sind zum Beispiel an acht Stellen, nämlich in zwei Reihen an je vier Stellen, maximal acht in **Fig. 6A** nicht dargestellte Abutment-Rohteile **17** montierbar. Die Platte **50** weist an jeder der acht Stellen ein Positionierungselement **14.2** auf, das dem ersten Positionierungselement **14** des Implantates **12** beziehungsweise dem Positionierungselement **14.1** des Manipulierbasisteiles **42** entspricht und das dazu bestimmt ist, mit dem Positionierelement **16** am Abutment-Rohteil **17** bzw. am Abutment **18** zusammenzuwirken. Ferner enthält die Platte **50** an jeder der acht Stellen eine nur in **Fig. 6B** sichtbare Gewindebohrung **52.** **Fig. 6B** stellt die Platte **50** der **Fig. 6A** im Schnitt dar, jedoch mit einem mittels einer Schraube **54** auf der Platte **50** befestigten Abutment-Rohteil **17** sowie mit zwei mittels weiterer Schrauben **54** auf der Platte **50** befestigbaren Abutment-Rohteilen **17** und mit einem aus einem bearbeiteten Abutment-Rohteil entstandenen, durch Lösung der Schraube **54** von der Platte **50** entfernten Abutment **18.** Solche Platten **50** sind bekannt. Die bisher auf ihnen hergestellten Abutments wiesen aber Achsen auf, die relativ zur Platte **50** parallel waren, während es in Wirklichkeit erforderlich ist, dass die verschiedenen Abutments relativ zur Platte **50** unterschiedlich gerichtete Achsen besitzen; die Richtungen dieser Achsen werden nach dem neuen Verfahren durch Verwendung der Hilfselemente beziehungsweise Vermessungsteile **44** durch die Erfassungseinrichtung **SCAN** festgestellt und bei der Herstellung der Abutments **18** mittels der CAD/CAM-Anlage **CC** berücksichtigt, so dass die derart erzeugten Abutments **18** achskonform montierbar sind.

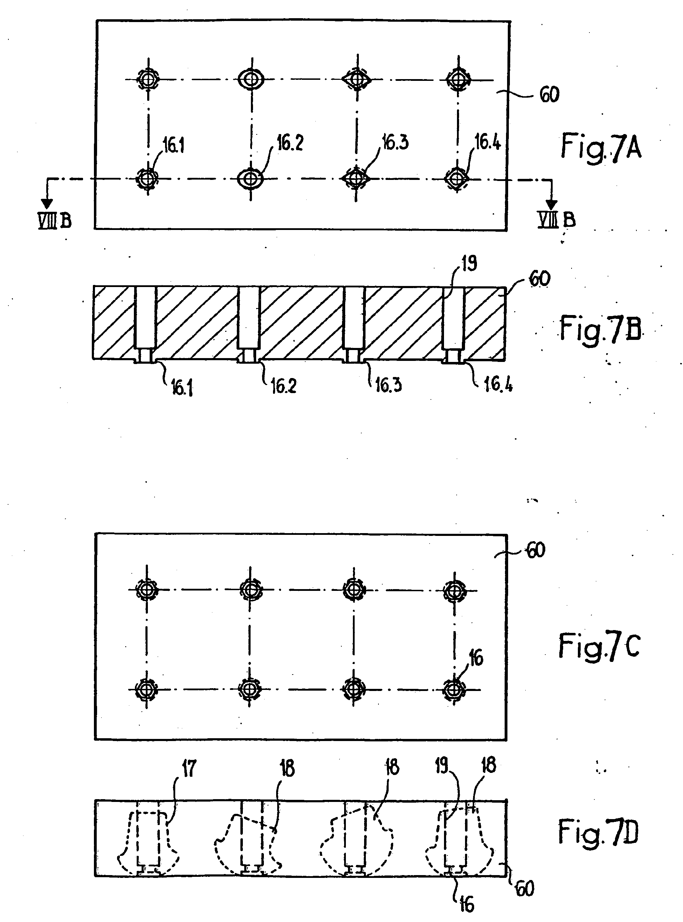

Eine weitere Möglichkeit zur Herstellung von Abutments ist in **Fig. 7A, 7B, 7C** und **7D** dargestellt. Hierbei werden die Abutments **18** direkt aus einer Platte 60 hergestellt. Diese Platte **60** besteht gesamthaft oder mindestens in den Bereichen, aus welchen die Abutments **18** erzeugt werden, aus dem Material der Abutments **18** selbst, dient aber auch als Vorrichtung zum Fixieren der entstehenden Abutments **18** während ihrer Herstellung. Die Platte **60** enthält bereits zum Beispiel an mehreren, im dargestellten Beispiel an acht, Stellen je ein Positionie rungselement 16, das auch am fertigen Abutment 18 dessen Positionierungselement 16 bilden wird. Die Positionierungselemente können beliebige Formen bilden, beispielsweise ein Hexagon, ein Oktogon, einen Rhombus, einen Ovalzylinder. Fig. 7A zeigt als Beispiel vier Varianten 16.1, 16.2, 16.3, 16.4 von rotationsverhindernden Positionierungselementen 16, die gemäss Fig. 7B alle nach Aussen ragen, obwohl im allgemeinen wie in Fig. 7C dargestellt alle Positionierungselemente 16 einer Platte 60 gleich ausgebildet sind. Die Positionierungselemente 16 der Platte 60 gemäss Fig. 7D sind dagegen im Inneren der Platte 60 angebracht. Die Platte 60 enthält auch die Durchgangsbohrungen 19 und die Auflage für einen Kopf der hier nicht dargestellten Schraube 54, die später zur Fixierung der fertigen Abutments 18 auf den Basisteilen bzw. Zahnresten benötigt werden. Wenn Abutments ohne Durchgangsbohrungen hergestellt werden müssen, so sind Platten 60 ohne die Bohrungen 19 zu verwendet. Aus der Platte 60 können somit acht Verbindungsstücke 18 hergestellt werden, die dazu bestimmt sind, auf je einem Basisteil befestigt zu werden. Die Formen der fertigen Abutments 18 sind in Fig. 7D mit gestrichelten Linien dargestellt.

Fig. 8A und Fig. 8B zeigen vier fertige Abutments 18, die aus einer Platte 60, hergestellt worden sind.

Fig. 9A und 9B zeigen, in analoger Darstellung wie Fig. 8A und 8B, wie die Herstellung des individuellen Zahnersatzteiles weiter rationalisiert werden kann, indem jeweils ein Abutment integral mit einem Gerüst, gegebenenfalls auch integral mit einer Verblendung, als Integralteil 24 ausgebildet und aus demselben Material in einem Bearbeitungsgang hergestellt werden. Besonders rationell ist es, mehrere Integralteile 24 aus einer Platte 70 herzustellen.

Die Abutments 18 und Integralteile 24, die aus Platten 60 bzw. 70 hergestellt werden, werden erst am Ende ihrer Bearbeitung von den Platten getrennt, falls die Platten 60 bzw. 70 direkt eingespannt werden. Alternativ können die Platten 60 bzw. 70 während der Bearbeitung der Abutments **18** bzw. Integralteile **24** mittels nicht dargestellter Hilfsschrauben, die in den Bohrungen 19 aufgenommen werden, auf einer nicht dargestellten Vorrichtung befestigt werden; dadurch sind die entstehenden Abutments **18** bzw. Integralteile **24** bis zum Ende der Bearbeitung gehalten. Abutments bzw. Integralteile aus Platten, die keine Bohrungen **19** aufweisen, können ggfs. nach einem ersten Teil der Bearbeitung eingespannt werden, damit sie bis zum Schluss der Bearbeitung gehalten sind.

Aus einer entsprechenden Platte könnte auch ein Integralteil mit drei Abschnitten hergestellt werden, wobei nur zwei Abschnitte zur Befestigung auf je einem Basisteil vorgesehen sind und dazu je ein Positionierungselement und eine Durchgangsbohrung aufweisen, während alle drei Abschnitte als Ersatz für insgesamt drei Zähne vorgesehen wären; hierzu müssten aber die Durchgangsbohrungen im erforderlichen Abstand angebracht werden.

Die Werkstoffe, aus welchen die einzelnen Elemente der Zahnersatzteile hergestellt werden, sind von entscheidender Bedeutung. Die verwendeten Werkstoffe sollen möglichst preisgünstig und mit vernünftigem Aufwand bearbeitbar sein, eine genügende Härte, Festigkeit und Elastizität und einen dem ursprünglichen Zahnmaterial ähnlichen thermischen Dilatationswert aufweisen, um nicht durch mechanische und thermische Beanspruchungen beeinträchtigt zu werden, chemisch resistent sein gegen alle in der Mundhöhle anzutreffenden körpereigenen, zu verspeisenden und bei der Zahnreinigung benutzten Stoffe, biokompatibel sein und die sichtbaren Bereiche der Zahnersatzteile sollten dem ursprünglichen Zahn optisch möglichst ähnlich sein. Ferner ist es erwünscht, dass die verschiedenen Bestandteile individueller Zahnersatzteile aus demselben Werkstoff hergestellt werden, um alle Probleme zu vermeiden, die sich durch Verwendung verschiedener Werkstoffe ergeben können, beispielsweise unterschiedliche thermische Dilatation, Bi-Material-Effekte wie Korrosion durch unterschiedliche Lage benachbarter Werkstoffe in der elektrischen Spannungsreihe und allzu unterschiedliche Lebensdauer.

Je nach Fall erweisen sich verschiedenen Werkstoffe als besonders geeignet zur Herstellung von Bestandteilen des individuellen Zahnersatzteiles. Geeignete Werkstoffe sind beispielsweise Metalle, Keramiken, Gläser und Kunststoffe. Die Bearbeitungsanlage, mittels welcher die CAM-Bearbeitung beispielsweise von Abutments durchgeführt wird, sollte daher zur Bearbeitung dieser Werkstoffe fähig sein.

Bezüglich der Techniken, welche zur Herstellung beziehungsweise Bearbeitung der verschiedenen Bestandteile der individuellen Zahnersatzteile eingesetzt werden, bestehen Einschränkungen. Insbesondere sind Gussverfahren und Erosionsverfahren, wie sie herkömmlicherweise häufig durchgeführt werden, zur Herstellung nicht unbedingt geeignet, da infolge grosser Temperaturdifferenzen Spannungen oder Materialveränderungen auftreten können. Aus medizinischen Gründen sind insbesondere unkontrollierbare Materialveränderungen zu vermeiden.

Bei Durchführung des erfindungsgemässen Verfahrens lassen sich alle oben erwähnten Anforderungen betreffend der Werkstoffe für individuelle Zahnersatzteile erfüllen. Insbesondere können weniger geeignete Gussverfahren vermieden werden und die Bearbeitungsanlage zur Durchführung der CAM-Prozedur kann alle in Frage kommenden Werkstoffe bearbeiten.

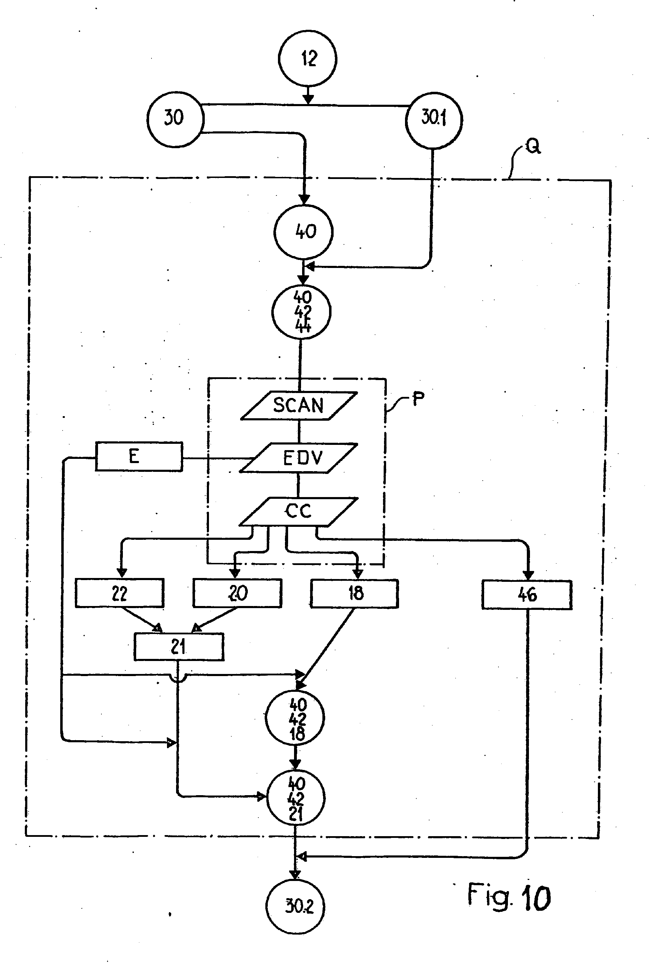

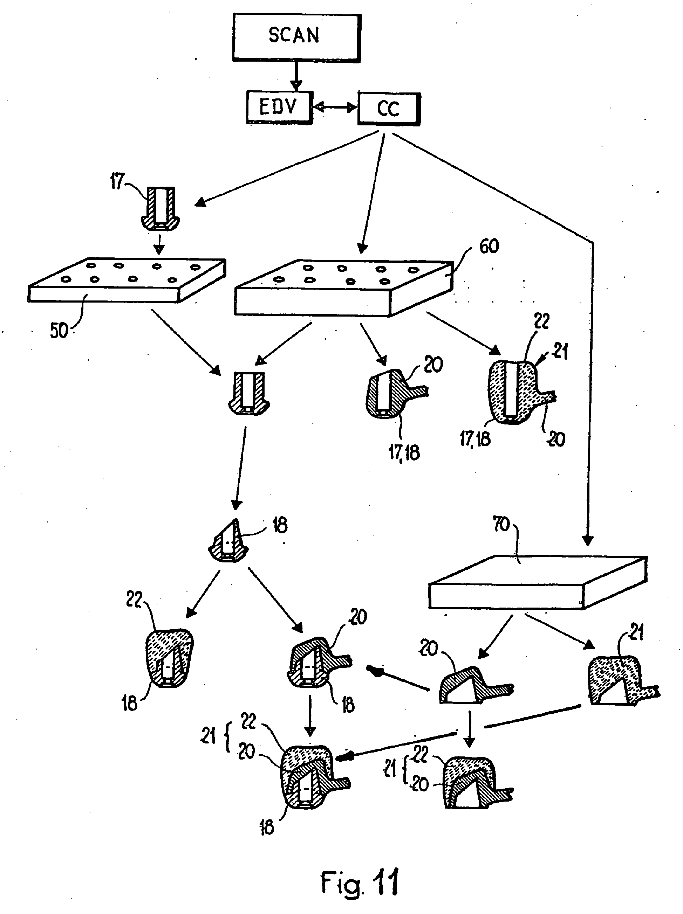

Die einzelnen Schritte eines Beispiels des gesamten Verfahrens der Herstellung und Eingliederung eines individuellen Zahnersatzteiles werden durch die schematischen Darstellungen der **Fig. 10** **und** **11** verdeutlicht.

In **Fig. 10** sind die erfindungsgemässen Schritte durch einen strichpunktierten Rahmen **Q** eingegrenzt. Das Arbeitsmodell **40,** je nach durchgeführten Verfahrensschritten mit den Bestandteilen **42, 18, 20, 22** des individuellen Zahnersatzes **10** gemäss der Stammanmeldung beziehungsweise mit dem Hilfselement **44** bestückt, ist stets als Kreis dargestellt, die einzelnen Teile der Einrichtung, welche zur Durchführung des Verfahrens benutzt werden, nämlich der Scanner **SCAN,** die EDV-Einheit **EDV** sowie die CAD/CAM-Anlage CC, sind als Parallelogramme dargestellt und durch einen Rahmen **P** zusammengefasst, und die aus der EDV-Einheit **EDV** und der CAD/CAM-Anlage CC resultierenden Bestandteile **12** (das heisst dem Implantat, das nur für den Zahnersatz 10 gemäss der Stammanmeldung benötigt wird) sowie den Bestandteilen **16, 18, 20, 22** (die für das individuelle Zahnersatzteil gemäss dem vorliegenden Teilgesuch) sowie das Hilfselement beziehungsweise Vermessungsteil **44** und Daten für deren Montage sind durch Rechtecke dargestellt. Ausserhalb des Rahmens **Q** angegebene Teile beziehungsweise Verfahrensschritte, nämlich der mit dem individuellen Zahnersatz **10** oder Zahnersatzteil zu versehende Kieferbereich 30 des Patienten, das danach gefertigte Negativmodell **32,** der mit den Implantaten **12** versehene Kieferbereich **30.1** und der mit dem individuellen Zahnersatz 10 bzw. Zahnersatzteil versehene Kieferbereich 30.2 des Patienten gehören nicht zur Erfindung. Im übrigen ist **Fig.10** selbsterklärend.

**Fig. 11** zeigt in anschaulicher Weise die verschiedenen Herstellungsvarianten sowie die Anfangs-, Zwischen- und Endstadien der Zahnersatzteile. Benutzt werden die Erfassungseinrichtung **SCAN,** die EDV-Anlage **EDV** und die CAD/CAM-Anlage CC.

Das Ausgangsmaterial für die Abutments **18** bilden entweder die Abutment-Rohteile **17,** die während ihrer Bearbeitung an den Platten **50** befestigt werden, oder die aus Abutment-Material bestehenden Platten **60.** Auf die Abutments **18** wird anschliessend die Suprakonstruktion **21** montiert; diese kann aus einem Teil, mit **21** bezeichnet, oder aus zwei Teilen, nämlich dem Gerüst **20** und der Verblendung **22,** bestehen.

Das Ausgangsmaterial für die Integralteile **24,** die entweder das Abutment und das Gerüst oder das Abutment und das Gerüst und die Verblendung integrieren, bildet die Platte **70**.

## Patentansprüche

1. Verfahren zur Herstellung eines Zahnersatzteiles **(18, 20, 21, 22),** welches dazu bestimmt ist, auf mindestens zwei Zahnresten positioniert und befestigt zu werden,
**dadurch gekennzeichnet,**
**dass** das Zahnersatzteil aus mindestens einem von mehreren an einer gemeinsamen Platte (50, 60, 70) angeordneten Zahnersatzteil-Rohteilen **(17)** durch eine CAD/CAM-Anlage **(CC)** aufgrund von Basis-Daten **(BAD)** hergestellt wird, welche die Geometrie des Zahnrestes definieren.

2. Verfahren nach Anspruch 1,
**dadurch gekennzeichnet,**
**dass** zur Bestimmung der Basis-Daten **(BD)** die dreidimensionale Geometrie des mit dem Zahnersatzteil zu versehenden Kieferbereiches direkt oder auf einem Arbeitsmodell durch eine Erfassungsvorrichtung **(SCAN)** erfasst wird.

3. Verfahren nach mindestens einem der Ansprüche 1 bis 2,
**dadurch gekennzeichnet,**
**dass** mehrere an derselben Platte **(50, 60, 70)** angeordnete Zahnersatzteil-Rohteile **(17)** nacheinander bearbeitet werden.

4. Verfahren nach mindestens einem der Ansprüche **1** bis **3,**
**dadurch gekennzeichnet,**
**dass** jedes Zahnersatzteil aus einem Abutment **(18)** oder aus einem Gerüst **(20)** oder aus einer Verblendung **(22)** oder aus einem Abutment **(18)** mit einem Gerüst **(20)** oder aus einer Suprakonstruktion **(21)** oder aus einem Integralteil **(24),** umfassend Abutment **(18),** Gerüst **(20)** und Verblendung **(22),** besteht.

5. Verfahren nach mindestens einem der Ansprüche **1** bis **4,**
**dadurch gekennzeichnet,**
**dass** die Zahnersatzteil-Rohteile während ihrer Bearbeitung einzeln an der als Vorrichtung dienenden Platte **(50)** befestigt werden.

6. Verfahren nach Anspruch **5,**
**dadurch gekennzeichnet,**
**dass** die Platte **(50)** Positionierungselemente **(14.2)** und die Zahnersatzteil-Rohteile komplementäre Positionierungselemente **(16)** aufweisen.

7. Verfahren nach mindestens einem der Ansprüche **1** bis **6,**
**dadurch gekennzeichnet,**
**dass** die Platte **(60, 70)** eine aus dem Material des Zahnersatzteil-Rohteils bestehende Platte ist, aus welcher die Zahnersatzteile hergestellt werden.

8. Verfahren nach Anspruch **7,**
**dadurch gekennzeichnet,**
**dass** die Platte die Positionierungselemente **(16)** und/oder Durchgangsbohrungen **(19)** der herzustellenden Zahnersatzteile aufweist.

9. Verfahren nach Anspruch **1**,
**dadurch gekennzeichnet,**
**dass** das Abutment unter Benutzung von Abutment-Daten (AD) aus einem einzelnen Abutment-Rohteil oder aus einem Standard-Abutment oder aus einer aus Abutment-Material bestehenden Platte hergestellt wird.

## Claims

1. A method for producing a dental prosthesis part (18, 20, 21, 22), which is intended for the purpose of being positioned and fastened on at least two tooth remnants,
**characterized in that**
the dental prosthesis part is produced from at least one of multiple dental prosthesis unfinished parts (17), which are situated on a common plate (50, 60, 70), via a CAD/CAM facility (CC) on the basis of base data (BAD), which define the geometry of the tooth remnant.

2. The method according to Claim 1,
**characterized in that,**
to determine the base data (BD), the three-dimensional geometry of the jaw area to be provided with the dental prosthesis part is scanned directly or on a working model by a scanning device (SCAN).

3. The method according to at least one of Claims 1 to 2,
**characterized in that**
multiple dental prosthesis unfinished parts (17), which are situated on the same plate (50, 60, 70), are processed in sequence.

4. The method according to at least one of Claims 1 to 3,
**characterized in that**
each dental prosthesis part comprises an abutment (18) or a framework (20) or a veneer (22) or an abutment (18) having a framework (20) or a superstructure (21) or an integral part (24), comprising abutment (18), framework (20), and veneer (22).

5. The method according to at least one of Claims 1 to 4,
**characterized in that**
the dental prosthesis unfinished parts are fastened individually during their processing on the plate (50) used as the device.

6. The method according to Claim 5,
**characterized in that**
the plate (50) has positioning elements (14.2) and the dental prosthesis unfinished parts have complementary positioning elements (16).

7. The method according to at least one of Claims 1 to 6,
**characterized in that**
the plate (60, 70) is a plate comprising the material of the dental prosthesis unfinished part, from which the dental prosthesis parts are produced.

8. The method according to Claim 7,
**characterized in that**
the plate has the positioning elements (16) and/or through holes (19) of the dental prosthesis parts to be produced.

9. The method according to Claim 1,
**characterized in that**
the abutment is produced employing abutment data (AD) from a single abutment unfinished part or from a standard abutment or from a plate comprising abutment material.

## Revendications

1. Procédé de fabrication d'un élément de prothèse dentaire **(18, 20, 21, 22)** qui est conçu pour être positionné et fixé sur au moins deux restes de dents,
**caractérisé en ce**
**que** l'élément de prothèse dentaire est fabriqué à partir d'au moins une parmi plusieurs ébauches **(17)** d'élément de prothèse dentaire disposées au niveau d'une plaque commune **(50, 60, 70),** par un dispositif de CAO/FAO **(CC),** sur la base de données de base **(BAD)** qui définissent la géométrie du reste de dent.

2. Procédé selon la revendication 1,
**caractérisé en ce**
**que**, pour déterminer les données de base **(BD),** on enregistre la géométrie en trois dimensions de la zone de mâchoire à munir de l'élément de prothèse dentaire, directement ou sur un modèle de travail par le biais d'un dispositif d'acquisition **(SCAN).**

3. Procédé selon au moins l'une des revendications 1 à 2,
**caractérisé en ce**
**que** plusieurs ébauches d'élément de prothèse dentaire **(17)** disposées sur la même plaque **(50, 60, 70)** sont usinées les unes après les autres.

4. Procédé selon au moins l'une des revendications 1 à 3,
**caractérisé en ce**
**que** chaque élément de prothèse dentaire se compose d'un pilier **(18)** ou d'une structure **(20)** ou d'une facette **(22)** ou d'un pilier **(18)** avec une structure **(20)** ou d'une superstructure **(21)** ou d'un élément intégral **(24),** comprenant un pilier **(18),** une structure **(20)** et une facette **(22).**

5. Procédé selon au moins l'une des revendications 1 à 4,
**caractérisé en ce**
**que** l'ébauche de l'élément de prothèse dentaire, pendant son usinage, est fixée individuellement au niveau de la plaque **(50)** servant de dispositif.

6. Procédé selon la revendication 5,
**caractérisé en ce**
**que** la plaque **(50)** présente des éléments de positionnement **(14.2)** et les ébauches d'élément de prothèse dentaire présentent des éléments de positionnement complémentaires.

7. Procédé selon au moins l'une des revendications 1 à 6,
**caractérisé en ce**
**que** la plaque **(60, 70)** est une plaque composée du matériau de l'ébauche d'élément de prothèse dentaire à partir de laquelle les éléments de prothèse dentaire sont fabriqués.

8. Procédé selon la revendication 7,
**caractérisé en ce**
**que** la plaque présente les éléments de positionnement **(16)** et/ou des perçages traversants **(19)** des éléments de prothèse dentaire à fabriquer.

9. Procédé selon la revendication 1,
**caractérisé en ce**
**que** le pilier, en utilisant des données de pilier **(AD),** est fabriqué à partir d'une ébauche de pilier individuelle ou d'un pilier standard ou d'une plaque composée d'un matériau de pilier.
